Europäisches Patentamt

European Patent Office

Office européen des brevets

(11) Publication number: **0 105 613**
**B1**

(12) **EUROPEAN PATENT SPECIFICATION**

(45) Date of publication of patent specification: **23.11.88**

(51) Int. Cl.⁴: **B 29 C 33/62,** A 61 B 19/04, C 08 J 7/04

(21) Application number: **83305099.0**

(22) Date of filing: **02.09.83**

(54) Dipped rubber article.

(30) Priority: **03.09.82 GB 8225200**
**30.11.82 US 445436**

(43) Date of publication of application:
**18.04.84 Bulletin 84/16**

(45) Publication of the grant of the patent:
**23.11.88 Bulletin 88/47**

(84) Designated Contracting States:
**AT BE CH DE FR GB IT LI LU NL SE**

(56) References cited:
FR-A-1 434 453
FR-A-1 453 817
FR-A-2 193 710
FR-A-2 297 910
US-A-3 813 695
US-A-3 959 554
US-A-4 100 309
US-A-4 143 109

(73) Proprietor: LRC PRODUCTS LIMITED
North Circular Road
London, E4 8QA (GB)

(72) Inventor: James, Michael Howard
Oakleigh House 12 West Common Way
Harpenden Hertfordshire (GB)
Inventor: Blackley, David Charles
100 Berkeley Avenue
Chesham Buckinghamshire HP5 2RS (GB)
Inventor: Podell, Howard Irwin
28 Beachfront Lane
New Rochelle New York, 10805 (US)
Inventor: Bratby, David Michael
18 Deborah Court Victoria Road
South Woodford London, E.18 (GB)
Inventor: Duck, Roger
29, Woodstock Road Upper Walthamstow
London, E.17 (GB)
Inventor: Goldstein, Albert
87, Glenwood Drive
Tinton Falls New Jersey 07724 (US)

(74) Representative: Austin, Hedley William et al
A.A. THORNTON & CO. Northumberland House
303-306 High Holborn
London WC1V 7LE (GB)

**Description**

The present invention is concerned with flexible rubber articles and, in particular, thin-walled rubber gloves of the kind used by surgeons.

Surgeon's gloves are difficult to don and to facilitate donning a powdered lubricant, such as particulate epichlorhydrin-treated maize starch, is conventionally applied to the inner surface of the gloves. There is a risk of such powdered lubricant escaping from the interior of the glove to contaminate the surgical field, the lubricant escaping either during donning or, as sometimes happens, if the glove is punctured during an operation.

Proposals have been made for polymeric lubricant coatings which are bonded to the inner surface of such gloves and which, because they are bonded, cannot escape from the glove.

Examples of such proposals are in U.S. Patents 4070713 and 4143109, which disclose gloves which have an inner layer of elastomeric material with particulate lubricant embedded therein, and U.S. Patents 3813695, 3856561 and 4302852, which disclose surgeon's gloves with various polymeric slip coatings bonded to the inner surface thereof.

U.S. Patent 3813695 ("the Podell patent") describes a surgeon's glove in which the glove material is formed of a laminate consisting of an outer layer of flexible material, for example rubber, and an inner layer of hydrophilic plastic material (such as a hydrogel polymer), the inner and outer layers being bonded together.

There are many known hydrogel polymers, examples of which are polyvinyl pyrrolidone, polyhydroxyethyl acrylate or methacrylate, polyhydroxypropyl acrylate or methacrylate, and copolymers of these with each other or with acrylic or methacrylic acid, acrylic or methacrylic esters or vinyl pyridine.

There are many disclosures of the coating of rubber articles, such as catheters and bathing caps, with such hydrogel polymers by dipping in a solution of a hydrophilic, hydrogel-forming polymer and curing the resulting polymer layer.

Examples of such disclosures include U.S. Patents 3326742, 3585103, 3607473, 3745042, 3901755, 3925138, 3930076, 3940533, 3966530, 4024317, 4110495 and 4125477, and British Patents 1028446 and 859297.

Whilst known hydrogel coatings on flexible rubber articles, such as surgeons gloves, provide a degree of lubricity to the skin contacting surface of such articles so that they can be donned without the need for a powdered lubricant, it is found that such lubricity is only manifested with respect to dry skin and is not present when the skin is damp or moist. Since surgeons prefer to don their gloves after "scrubbing up", without fully drying their hands, their hands are distinctly damp. We have found that most hydrogel polymers used as bonded inner layers in surgeons' gloves, as suggested in the Podell patent, give totally inadequate lubricity as regards damp hands. We have now developed a treatment for the hydrogel-coated surface of such rubber articles which provides good lubricity with respect to damp skin.

We have also found that hydrogels based on certain 2-hydroxyethyl methacrylate polymers provide superior lubricity with respect to dry skin.

According to the present invention, therefore, there is provided a flexible rubber article having a lubricating layer formed from a hydrogel polymer bonded thereto so as to provide a skin-contacting surface of said article, characterised in that a surfactant material or a long chain fatty amine has been applied to said skin-contacting surface so as to substantially improve the lubricity of said surface with respect to damp skin.

Such surfactants and fatty amines improve the lubricity with respect to damp skin for a wide range of hydrogel polymers as well as the specific 2-hydroxyethyl methacrylate copolymers referred to above. The surfactants and fatty amines used are preferably bactericidal or bacteriostatic. The surfactant used is preferably ionic (it may sometimes be preferred for the surfactant to have opposite polarity to that of the hydrogel-forming polymer); cationic surfactants are particularly preferred. Such cationic surfactants may have a free anion, such as a halide ion, or the anion may be part of the molecular structure of the surfactant (that is, the latter has a betaine structure).

Preferred cationic surfactants are quaternary ammonium compounds having at least one 6—18C hydrocarbyl (alkenyl or alkyl) group; a preferred hydrocarbyl group is a hexadecyl group. It is further preferred that the hydrocarbyl group is attached to a quaternary nitrogen atom which is part of a heterocyclic ring (such as a pyridine, morpholine or imidazoline ring).

Most preferred cationic surfactants are hexadecyl trimethyl ammonium chloride, N-lauryl pyridinium chloride, N-cetyl pyridinium chloride, the corresponding bromides, or a hydroxyethyl heptadecenyl imidazoline salt, all of which significantly improve the lubricity with respect to damp skin without adversely affecting lubricity with respect to dry skin.

Non-ionic and anionic surfactants may be used instead of cationic surfactants; examples of suitable non-ionic surfactants include ethylene oxide condensates (such as polyethylene oxide, ethylene oxide-polypropylene glycol condensates, and polyglycol-polyamine condensates). An example of a suitable anionic surfactant is sodium lauryl sulphate.

When a (neutral) fatty amine is used, this preferably has a 6—18C hydrocarbyl group (such as a hexadecyl group) attached to the nitrogen atom. A preferred such amine is N,N-dimethyl hexadecylamine (which is commercially available as Armeen 16D).

The use of cationic surfactants serves to inhibit bacterial growth when the layer formed from the hydrogel polymer is in contact with the skin; this is an advantage for surgeon's gloves because, as mentioned above, these are sometimes punctured during surgical procedures, and any bacteria which have grown on the surgeon's skin since commencement of the operation may be released into the surgical field.

The surfactant or fatty amine is generally used in the form of a solution, such as an aqueous solution (typically an aqueous solution containing at elast 0.2% by weight, up to, for example, 2% by weight) of a cationic surfactant as just mentioned.

The article according to the invention is preferably treated with a silicone liquid so as to reduce the surface tack on any surfaces not coated with a layer formed from the hydrogel polymer; this treatment is preferably carried out at the same time as treatment with a surfactant as mentioned above. It is preferred that treatment with a silicone (such as medical grade polydimethyl siloxane) is carried out with a bath containing at least 0.05% by weight of silicone (for example, 0.05 to 0.4% by weight).

As indicated above, we have also discovered a range of 2-hydroxyethyl methacrylate (HEMA) copolymers which provide hydrogels imparting superior lubricity with respect to dry skin and the hydrogel coating of the flexible rubber article according to the invention is preferably formed from such a copolymer. These copolymers are copolymers of 2-hydroxyethyl methacrylate (HEMA) with methacrylic acid (MAA) or with 2-ethylhexyl acrylate (EHA), or a ternary copolymer of HEMA, MAA and EHA, said copolymer having a composition within the area ABCDEF in the attached ternary composition diagram (see divisional Application 86105456.7. The area ABCDEF is believed to cover substantially all of the hydrogel-forming area of the composition diagram, apart from HEMA homopolymer and HEMA copolymers with up to 5% EHA and/or MAA.

The composition is preferably within area ABXYZ, more preferably within area PQRI. It is most preferred that the composition is within area IJKL.

In some embodiments, such a copolymer may contain HEMA and MAA in a molar ratio of at least 1:1 (such as 1 to 10:1) or HEMA and EHA in a molar ratio of at least 2.5:1 (such as 2.5 to 10:1).

The copolymer, which is preferably prepared by solution polymerization (bulk polymerisation is less satisfactory), may be a binary copolymer of HEMA and MAA or EHA, or it may be a ternary copolymer of these monomers with EHA; a preferred such terpolymer has a monomer molar ratio of HEMA to (MAA+EHA) of 67:33 to 90:10 (that is 2 to 9:1) and a molar ratio of EHA to (HEMA+MAA) of 5:97 to 20:80 (that is 1: about 20 to 4).

Minor amounts of further monomers which do not impair the properties of the copolymer may be used in addition. A mixture of such copolymers can be employed, either with other such copolymers or with minor amounts of other polymers which do not impair the properties of the hydrogel-forming polymer.

It is to be noted that the EHA is of a hydrophobic nature and assists in the bonding of the hydrogel polymer to the (hydrophobic) rubber substrate. EHA also serves as a plasticiser and increases the flexibility of the layer formed from the hydrogel polymer (which is bonded to a flexible rubber substrate). EHA may accordingly be replaced by an alkyl acrylate or methacrylate which acts in a corresponding manner.

The MAA serves to furnish cross-linking sites in the copolymer; the methyl group in MAA may influence the copolymer by

(i) modifying the reactivity of the carboxyl groups and thus influencing the rate and degree of cross-linking, and

(ii) by modifying the rate at which MAA is incorporated into the polymer backbone and hence the distribution of cross-linking sites along the polymer chain.

Copolymers as described above provide better lubricity with respect to dry skin than any other hydrogel-forming polymer of the many we have evaluated.

The rubber used in the article according to the present invention may be a natural or synthetic rubber; natural rubber is preferred. It is also preferred that the article according to the invention should be formed (prior to bonding of the layer formed from hydrogel polymer thereto) by dipping of a rubber latex. The hydrogel layer is preferably applied to the rubber before vulcanisation thereof; this has the surprising result that the skin-contacting surface has a much cooler feel (see divisional Application 86105454.2). This may be because there is greater vapour transmission through the final coated article. The hydrogel-forming polymer solution, which preferably contains a curing agent therefor, is preferably applied by dipping the rubber (on a former) in the solution.

An article according to the invention can be produced by a process comprising the steps of:

(a) forming a rubber article by dipping a suitably shaped former in a rubber latex;

(b) leaching the rubber article on the former in hot water,

(c) priming the rubber surface of the article on the former, for example, by means of a dilute acid,

(d) rinsing the primed surface in water or aqueous alkali,

(e) dipping said article, while still on the former, in a solution of a hydrophilic, hydrogel-forming polymer and a curing agent therefor,

(f) heat drying the resulting coating such that the resulting hydrogel polymer is bonded to said rubber,

(g) vulcanising the rubber and simultaneously curing the polymer by application of heat,

(h) stripping the resulting article from the former,

3

(i) applying a solution of the surfactant material containing silicone to the article (for example, by tumbling in such a solution), optionally after washing, and

(j) heating the resulting coating of surfactant material so as to fix the slip properties of the coating.

Our tests have shown that, following step (j), the damp skin slip properties and the dry skin slip properties of the coating formed from the hydrogel polymer are advantageously not impaired by subsequent washing (that is, surfactant material is not leached out to any substantial extent on washing of the coating article).

The application of the solution of surfactant material provides a substantially tack-free outer surface (that is, the surface not coated with hydrogel polymer), in addition to the inner surface, which is, of course, advantageous.

The rubber surface to which the hydrogel polymer is bonded may also be primed by dipping in, for example, a solution of an aluminium salt after priming with dilute acid.

It is a feature of the present invention that the production of the dipped rubber article, leaching, priming, application of hydrogel polymer layer, and vulcanisation of the rubber and curing of the polymer can all be carried out in a continuous operation.

The procedure of forming coatings on dipped rubber articles by multiple dipping of a suitably shaped former is not in itself novel and is described, for example, in French patent 1,434,453. This patent describes *inter alia* the application of a slippery finish to a rubber article by a process which comprises:

1. Coating a suitably shaped former with a coagulant by dipping it into a coagulant composition,
2. drying the coagulant coating on the former,
3. dipping the former into a pre-vulcanised rubber latex,
4. removing the former from the latex,
5. drying the latex coating in air,
6. leaching coagulant from the latex coating by dipping the former into hot water,
7. removing the former from the wash liquor and dipping it in a bath of the slippery finish coating,
8. removing the former from the finish coating bath,
9. allowing the coating to dry in air,
10. pass the former into an oven heated to a temperature of 88 to 93°C to dry and vulcanise the materials, and
11. stripping the finished article from the former.

The present invention has been described primarily, with reference to surgeons' gloves; it is, however, applicable to other skin- or tissue-contacting flexible rubber articles, such as condoms, gloves used by doctors and veterinary surgeons for examination purposes (such gloves being often donned with dry hands), catheters, urethers, sheets and sheath-type incontinence devices.

When the present invention is used for articles such as urethers and catheters, the layer formed from hydrogel polymer is provided on the outer surface (this being the skin-contacting surface); for condoms the layer formed from hydrogel polymer may be provided on the inner surface and/or on the outer surface.

In order that the present invention may be more fully understood, the following Examples and Comparative Examples are given by way of illustration only.

Example 1

A thin dipped surgeons glove of natural rubber latex was leached with sulphuric acid, rinsed, primed by dipping in aluminium sulphate solution, dried out completely and then dipped into a 4% alcoholic solution of a copolymer of 2-hydroxyethyl methacrylate (HEMA) and methacrylic acid (MAA) in a 1:1 molar ratio, followed by drying. The solution contained, in addition to the copolymer, 5 parts per hundred of partially methylated melamine-formaldehyde resin (as cross-linking agent) and 0.5 parts per hundred of paratoluene sulphonic acid (as catalyst).

The rubber was then vulcanised, after which the lubricity with respect to dry skin was subjectively evaluated on a scale of 1 to 5, in which:

1 means that the film is sticky
2 means that poor slip is obtained
3 means that moderate slip is obtained
4 means that quite good slip is obtained
5 means that excellent slip is obtained (comparable to the use of a powdered surface).

The dry skin lubricity number was 5; the coating adhered satisfactorily to the rubber and no visible flaking was observed.


Examples 2 to 11

Example 1 was repeated, except that the copolymer was replaced by the polymers indicated in the following Table 1:

TABLE 1

| Example No. | Polymer | Molar ratio of monomers | Dry skin lubricity number |
|---|---|---|---|
| 2 | HEMA/MAA | 2:1 | 5 |
| 3 | HEMA/MAA | 5:1 | 5 |
| 4 | HEMA/MAA | 10:1 | 5 |
| 5 | HEMA/EHA | 2.5:1 | 5 |
| 6 | HEMA/EHA | 4:1 | 5 |
| 7 | HEMA/EHA | 5:1 | 5 |
| 8 | HEMA/EHA | 10:1 | 5 |
| 9 | HEMA/MAA/EHA | 10:1:0.5 | 5 |
| 10 | HEMA/MAA/EHA | 5:1:1.2 | 5 |
| 11 | HEMA/MAA/BA | 10:1:0.5 | 5 |
| Comparative 1 | HEMA/AA | 2:1 | 4 |
| Comparative 2 | HEMA/AA | 1:1 | 4 |
| Comparative 3 | HEMA/MMA | 2:1 | 4 |
| Comparative 4 | HEMA/MMA | 1:1 | 4 |
| Comparative 5 | HEMA/BA | 5:1 | 4 |
| Comparative 6 | HEMA/BA | 2:1 | 3 |
| Comparative 7 | HEMA/MA | 2:1 | 4 |
| Comparative 8 | HEMA/IA | 2:1 | 4 |
| Comparative 9 | HEMA/EHA | 2:1 | 4 |
| Comparative 10 | HEMA/EHA | 1:1 | 4 |
| Comparative 11 | MMA/VPd | 1:1 | 3 |
| Comparative 12 | HEMA | — | 3—4 |
| Comparative 13 | HEA | — | 3—4 |
| Comparative 14 | VPd | — | 2 |
| Comparative 15 | HPMA | — | 3—4 |
| Comparative 16 | HEMA/HEA | 1:1 | 4 |
| Comparative 17 | HEMA/VPd | 1:1 | 3—4 |
| Comparative 18 | HEMA/HPMA | 1:1 | 4 |
| Comparative 19 | HEA/HPMA | 1:1 | 4 |
| Comparative 20 | HEMA/VPy | 9:1 | 4—5 |

# EP 0 105 613 B1

In the above Table, the abbreviations have the following meanings:

| | |
|---|---|
| EHA | 2-ethylhexyl acrylate |
| BA | butyl acrylate |
| AA | acrylic acid |
| MMA | methyl methacrylate |
| MA | methyl acrylate |
| IA | itaconic acid |
| VPd | N-vinyl pyrrolidone |
| HEA | hydroxyethyl acrylate |
| HPMA | hydroxypropyl methacrylate |
| VPy | vinyl pyridine (quaternised) |

It will be seen that the dry skin lubricity for each of the Examples according to the invention was better than that obtained in any of the Comparative Examples (the only comparative sample approaching the lubricity of the samples according to the invention was that of Comparative Example 20, when a quaternised copolymer was used).

In each of the Examples according to the invention, the coating adhered satisfactorily with at most very slight flaking. This also applied to the Comparative Examples, except Comparative Example 14 (where the coating was washed off on wet-stripping).

The dry frictional force and the coefficient of friction for the glove of Example 6, the glove of Example 10 and for a conventional powdered glove are given in the following Table 2.

TABLE 2

| Glove | Dry frictional force | Coefficient of friction |
|---|---|---|
| Example 6 | 48.7 g (0.48 N) | 0.20 |
| Example 10 | 53.1 g (0.52 N) | 0.218 |
| Conventional powdered | 78.5 g (0.77 N) | 0.323 |

The damp skin lubricity number was 2 for Examples 1 to 11 and Comparative Examples 2 to 10, 12, 13, and 15 to 20, and 1 for Comparative Examples 1, 11 and 14.

Examples 12 to 21

Samples prepared as in Example 10 were post-treated by dipping in solutions of various materials, as identified in the following Table 3.

6

TABLE 3

| Example No. | Material | Concentration of solution | Damp skin lubricity number |
|---|---|---|---|
| 12 | N-cetylpyridinium chloride (N-CPC) | 5% | 3—4 |
| 13 | —do— | 1% | 3—4 |
| 14 | Sodium lauryl sulphate | 5% | 3 |
| 15 | Sodium lauryl sulphate | 1.0% | 3 |
| 16 | —do— | 0.5% | 3 |
| 17 | —do— | 0.1% | 2—3 |
| 18 | N,N-dimethyl hexadecylamine | 1% | 3—4 |
| 19 | Ethylene oxide-polypropylene glycol condensate | 1% | 3 |
| 20 | Distearyl dimethyl ammonium chloride | 1% | 3 |
| 21 | Hexadecyl trimethyl ammonium chloride | 1% | 3—4 |

In each case, the dry slip was substantially unimpaired.

Examples 22 to 26

Example 12 was repeated, using solutions containing various proportions of N-CPC and also 0.3% medical grade polydimethyl siloxane, as indicated in the following Table 4.

TABLE 4

| Example No. | Percentage N-CPC | Damp skin lubricity number |
|---|---|---|
| 22 | 0.1 | 3 |
| 23 | 0.25 | 3—4 |
| 24 | 0.50 | 4 |
| 25 | 1.0 | 4 |
| 26 | 2.0 | 4 |

Similar results to those of Example 22 were obtained when the percentage of polydimethyl siloxane was 0.05%.

Example 27

A series of hand-shaped formers were dipped into a natural rubber latex to produce a thin rubber layer on each former. The rubber layer was leached in hot water and then primed by dipping in dilute sulphuric acid, rinsed, dipped into a caustic soda bath of pH 10.5 containing hydrogen peroxide in an amount sufficient to react with hydrogen sulphide formed in the priming stage. The rubber, still on the formers, was then dipped into a 4% ethanolic solution of a HEMA/MAA/EHA terpolymer with a monomer molar ratio of 5:1:1.2, the solution also containing 5 to 15 parts per hundred (based on the weight of polymer) of partially

7

methylated melamine-formaldehyde resin available commercially as Cymel® 373 (as cross-linking agent) and 0.5 to 1.5 parts per hundred (on the same basis) of para-toluene sulphonic acid (as catalyst).

The rubber was then vulcanised and the polymer simultaneously cured (the temperature being raised from 80 to 150°C over 25 minutes during vulcanisation), the resulting gloves being stripped from the formers.

The stripped gloves were washed with water and then tumbled in an aqueous solution containing 0.75% by weight of N-cetylpyridinium chloride, the solution also containing 0.05% by weight of emulsified silicone. The gloves were finally tumbled dry at 65°C for 75 minutes.

The resulting gloves had a dry skin lubricity number of 5 and a wet skin lubricity number of 4 on their polymer-coated surfaces (used as the insides of the gloves).

No allergenic or irritant reaction to the gloves was reported, even when the gloves were worn by surgeons with hypersensitive skin.

Example 28

Example 1 was repeated except that the copolymer was replaced by a copolymer having the following molar percentage: 80% HEMA, 10% MAA, 10% EHA. A dry skin lubricity number of 5 was obtained.

Example 29

A rubber-coated porcelain mandrel was dipped for several minutes in water at above 70°C, rinsed in running water, and dipped in 2% sulphuric acid at 40°C. The coated mandrel was then dipped for (neutralisation) in dilute caustic (pH 9—10) and then dipped in water wash tanks at 40°C. The coated mandrel was then coated with a 10% solution in ethanol of a terpolymer as used in example 10, the solution containing 10% by weight of Cymel® 370 (cross-linking agent) and 1% by weight of p-toluenesulphonic acid.

The coated mandrel was heated in an oven for 30 minutes with temepratures rising to 105°C.

The glove was stripped from the mandrel and immersed for 15 minutes in an aqueous dispersion of 0.05% to 35% Silicone medical grade emulsion DC365 (Dow Corning brand) containing 0.5% Cetylpyridinium chloride. After draining, the glove was heated and dried in an oven for 30 minutes at 70°C.

The outer surface of the glove was tack-free; the inner coated surface was hydrophilic, had a high degree of slip and was readily donned on a dry hand (a dry skin lubricity number of 5).

The moisture transmission properties at 25°C (100% RH) of the resulting glove and an otherwise similar, but uncoated (control), glove are set out in the following Table 5.

TABLE 5

| Sample | Rate of moisture transmission (gm/m²/mm/24 hrs.) |
|---|---|
| According to the invention | 7.86 |
| Control | 4.22 |

Examples 30 to 37

Example 1 was repeated, except that the copolymer was replaced by the polymers indicated in the following Table 6.

8

# EP 0 105 613 B1

TABLE 6

| Example No. | Molar percentage HEMA | Molar percentage MAA | Molar percentage EHA |
|---|---|---|---|
| 30 | 70 | 20 | 10 |
| 31 | 60 | 10 | 30 |
| 32 | 60 | 20 | 20 |
| 33 | 60 | 30 | 10 |
| 34 | 50 | 30 | 20 |
| 35 | 40 | 30 | 30 |
| 36 | 40 | 40 | 20 |
| 37 | 30 | 60 | 10 |

In each case, a dry skin lubricity number of 5 was obtained.

Examples 38 to 53

Example 12 was repeated, using solutions of various surfactants instead of N-CPC. The results are summarised in the following Table 7.

Example 54

Example 1 was repeated, except that the copolymer was replaced by a copolymer having the following molar percentage: 70% HEMA, 10% MAA, 10% EHA. A dry skin lubricity number of 5 was obtained.

9

EP 0 105 613 B1

TABLE 7

| Example No. | Surfactant | Concentration of solution | Damp skin lubricity number |
|---|---|---|---|
| 38 | Octadecyl trimethyl-ammonium bromide | 1.0% | 3 |
| 39 | Cetyl pyridinium bromide | 1.0% | 3—4 |
| 40 | 2-oleyl-1-(ethylbetaoxy propionic acid) imidazoline | 1.0% | 3 |
| 41 | Lauryl pyridinium chloride | 1.0% | 3—4 |
| 42 | Oxyethyl alkyl ammonium phosphate | 1.0% | 3 |
| 43 | 10—18C alkyl dimethyl benzyl ammonium chloride | 1.0% | 3 |
| 44 | Polypropoxy quaternary ammonium acetate | 1.0% | 3 |
| 45 | 12—14C alkylbetaine | 1.0% | 3 |
| 46 | Coconut imidazoline betaine | 1.0% | 3 |
| 47 | An amine oxide ethoxylate (Empigen DY) | 1.0% | 3 |
| 48 | N-cetyl-N-ethyl morpholinium ethosulphate | 1.0% | 3 |
| 49 | Distearyl dimethylammonium chloride | 1.0% | 3 |
| 50 | Pluronic F38 (an ethylene oxide-polypropylene glycol of mol. wt. 5020) | 0.15% | 3 |
| 51 | Polyethylene oxide (mol. wt. 400000) | 1.0% | 3 |
| 52 | Dodecylbenzyl-hydroxymethyl dimethyl chloride | 1.0% | 3 |
| 53 | Cetyl stearyl ethylene oxide condensate (20 ethylene oxide units). | 5% | 3 |

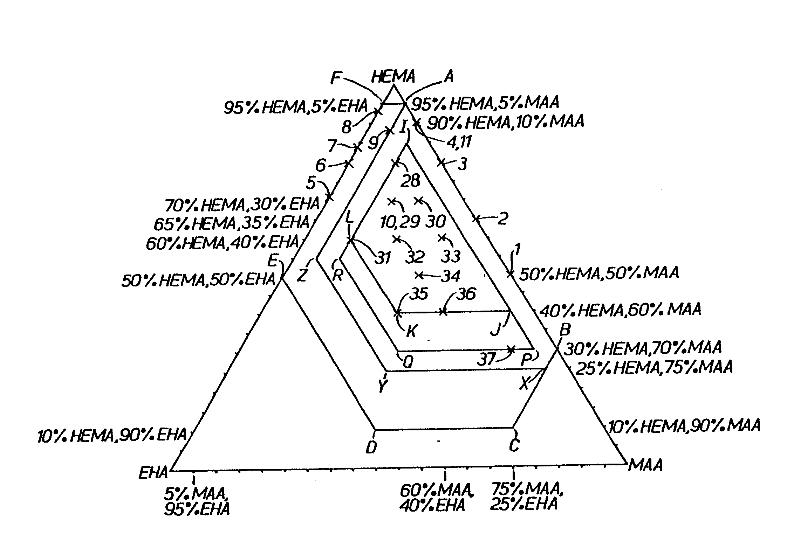

## Claims

1. A flexible rubber article having a lubricating layer formed from a hydrogel polymer bonded thereto so as to provide a skin-contacting surface of said article, characterised in that a surfactant material or a long chain fatty amine has been applied to said skin-contacting surface so as to substantially improve the lubricity of said surface with respect to damp skin.

2. An article according to claim 1, in which the surfactant material comprises an ionic surfactant.

3. An article according to claim 2, in which said surfactant is cationic.

4. An article according to claim 3, in which said surfactant is a quaternary ammonium compound having at least one 68—18C hydrocarbyl group.

5. An article according to claim 4, in which said hydrocarbyl group is attached to a quaternary nitrogen atom which is part of a heterocyclic ring.

6. An article according to claim 5, in which the heterocyclic ring is pyridine, morpholine or imidazoline.

7. An article according to claim 6, in which the surfactant is an N-lauryl or N-cetyl pyridinium salt, or a hydroxyethyl heptadecenyl imidazoline salt.

8. An article according to claim 4, in which said surfactant is hexadecyl trimethyl ammonium chloride.

9. An article according to any of claims 1 to 8, in which the hydrogel polymer is a copolymer of 2-hydroxyethyl methacrylate with methacrylic acid and/or with 2-ethylhexyl acrylate, said copolymer having a composition within the area ABCDEF of the attached ternary composition diagram.

10. An article according to claim 9, in which the 2-ethylhexyl acrylate is replaced by another alkyl acrylate or an alkyl methacrylate having an equivalent effect.

10

11. An article according to claim 9, in which the copolymer has a composition within the area PQRI of the attached ternary composition diagram.

12. A process for producing an article according to any of claims 1 to 11, which comprises the steps of:

(a) forming a rubber article by dipping a suitably shaped former in a rubber latex,

(b) leaching the rubber article on the former in hot water,

(c) priming the rubber surface of the article on the former by means of a dilute acid,

(d) rinsing the primed surface in water or aqueous alkali,

(e) dipping said rubber article, while still on the former, in a solution of a hydrophilic, hydrogel-forming polymer and a curing agent therefor,

(f) heat drying the resulting coating such that the resulting hydrogel polymer is bonded to said rubber,

(g) vulcanising the rubber and simultaneously curing the polymer by the application of heat,

(h) stripping the resulting article from the former,

(i) applying a solution of the surfactant material containing silicone to said coating of hydrogel polymer, and

(j) heating the resulting coating of surfactant material so as to fix the slip properties of the coating.

**Patentansprüche**

1. Flexibler Gummigegenstand mit einer aus einem daran gebundenen Hydrogel-Polymer gebildeten Gleitschicht, um eine Hautkontaktoberfläche des Gegenstands vorzusehen, dadurch gekennzeichnet, daß ein Surfactantmaterial oder ein langkettiges Fettamin auf die Hautkontaktoberfläche aufgetragen worden ist, um die Gleitfähigkeit der Oberfläche gegenüber feuchter Haut wesentlich zu verbessern.

2. Gegenstand nach Anspruch 1, wobei das Surfactantmaterial ein ionisches Surfactant umfaßt.

3. Gegenstand nach Anspruch 2, wobei das Surfactant kationisch ist.

4. Gegenstand nach Anspruch 3, wobei das surfactant eine quaternäre Ammoniumverbindung mit mindestens einer 6—18C-Kohlenwasserstoffgruppe ist.

5. Gegenstand nach Anspruch 4, wobei die Kohlenwasserstoffgruppe an ein quaternäres Stickstoffatom gebunden ist, das Teil eines heterocyclischen Rings ist.

6. Gegenstand nach Anspruch 5, wobei der heterocyclische Ring Pyridin, Morpholin oder Imidazolin ist.

7. Gegenstand nach Anspruch 6, wobei das Surfactant ein N-Lauryl- oder N-Cetylpyridiniumsalz oder ein Hydroxyethylheptadecenylimidazolinsalz ist.

8. Gegenstand nach Anspruch 4, wobei das Surfactant Hexadecyltrimethylammoniumchlorid ist.

9. Gegenstand nach wenigstens einem der Ansprüche 1 bis 8, wobei das Hydrogel-Polymer ein Copolymer von 2-Hydroxyethylmethacrylat mit Methacrylsäure und/oder mit 2-Ethylhexylacrylat ist, wobei das Copolymer eine Zusammensetzung innerhalb des Bereichs ABCDEF des beiliegenden ternären Zusammensetzungsdiagramms besitzt.

10. Gegenstand nach Anspruch 9, wobei das 2-Ethylhexylacrylat ersetzt ist durch ein anderes Alkylacrylat oder ein Alkylmethacrylat mit eine äquivalenten Wirkung.

11. Gegenstand nach Anspruch 9, wobei das Copolymer eine Zusammensetzung innerhalb des Bereichs PQRI des beiliegenden ternären Zusammensetzungsdiagramms besitzt.

12. Verfahren zur Herstellung eines Gegenstands nach mindestens einem der Ansprüche 1 bis 11, umfassend die Stufen:

a) Bildung eines Gummigegenstands durch Eintauchen eines geeignet geformten Formers in einen Gummilatex,

b) Auslaugen des Gummigegenstands auf dem Former in heißem Wasser,

c) Grundieren der Gummioberfläche des Gegenstands auf dem Former mittels einer verdünnten Säure,

d) Spulen der grundierten Oberfläche in Wasser oder wäßrigem Alkali,

e) Eintauchen des Gummigegenstands, während er sich noch auf dem Former befindet, in eine Lösung aus einem hydrophilen Hydrogel bildenden Polymer und einem Härtungsmittel hierfür,

f) Wärmetrocknen der resultierenden Beschichtung, so daß das resultierende Hydrogel-Polymer an dem Gummi gebunden wird,

g) Vulkanisieren des Gummis und gleichzeitiges Härten des Polymers durch Anwendung von Wärme,

h) Abstreifen des resultierenden Gegenstands von dem Former,

i) Auftragen einer Lösung des Surfactantmaterials, enthaltend Silikon, auf die Beschichtung aus dem Hydrogel-Polymer und

j) Erhitzen der resultierenden Beschichtung aus dem Surfactantmaterial, um die Gleiteigenschaften der Beschichtung zu fixieren.

**Revendications**

1. Un article de caoutchouc souple sur lequel on a fixé une couche lubrifiante formée à partir d'un polymère un hydrogel et constituant la surface de contact de l'article avec la peau, caractérisé en ce que, sur ladite surface de contact avec la peau, et afin d'améliorer dans une mesure importante les propriétés de glissement de ladite surface sur la peau humide, on a appliqué un agent tensioactif ou une amine grasse à longue chaîne.

2. Un article selon la revendication 1, dans lequel l'agent tensioactif est un agent tensioactif ionique.

3. Un article selon la revendication 2, dans lequel l'agent tensioactif est cationique.

4. Un article selon la revendication 3, dans lequel l'agent tensioactif est un composé d'ammonium quaternaire contenant au moins un groupe hydrocarboné en $C_6$—$C_{18}$.

5. Un article selon la revendication 4, dans lequel le groupe hydrocarboné est fixé à un atome d'azote quaternaire faisant partie d'un noyau hétérocyclique.

6. Un article selon la revendication 5, dans lequel le noyau hétérocyclique est un noyau de pyridine, de morpholine ou d'imidazoline.

7. Un article selon la revendication 6, dans lequel l'agent tensioactif est un sel de N-lauryl- ou de N-cétyl-pyridinium ou un sel d'hydroxéthyl-heptadécényl-imidazoline.

8. Un article selon la revendication 4, dans lequel l'agent tensioactif est le chlorure d'hexadécyl-triméthyl-ammonium.

9. Un article selon l'une quelconque des revendications 1 à 8, dans lequel le polymère en hydrogel est un copolymère du méthacrylate de 2-hydroxyéthyle et de l'acide méthacrylique et/ou de l'acrylate de 2-éthylhexyle, ce copolymère ayant une composition qui se situe dans l'aire ABCDEF du diagramme ternaire de composition annexé.

10. Un article selon la revendication 9, dans lequel l'acrylate de 2-éthylhexyle est remplacé par un autre acrylate ou méthacrylate d'alkyle ayant un effet équivalent.

11. Un article selon la revendication 9, dans lequel le copolymère a une composition située à l'intérieur de l'aire PQRI du diagramme ternaire de composition annexé.

12. Un procédé pour la préparation d'un article selon l'une des revendications 1 à 11, qui comprend les stades opératoires suivants:

(a) la formation d'un article de caoutchouc par immersion d'une forme appropriée dans un latex de caoutchouc,

(b) le lavage de l'article de caoutchouc, sur la forme, dans l'eau chaude,

(c) l'application sur la surface de caoutchouc de l'article, sur la forme, d'une couche d'apprêt à l'aide d'un acide dilué,

(d) le rinçage de la surface apprêtée dans l'eau ou un alcali aqueux,

(e) l'immersion de l'article de caoutchouc, toujours sur la forme, dans une solution d'un polymère hydrophile formant un hydrogel et d'un agent réticulant de ce polymère,

(f) le séchage à chaud du revêtement appliqué provoquant la fixation du polymère en hydrogel sur la caoutchouc,

(g) la vulcanisation du caoutchouc avec réticulation simultanée du polymère par application de chaleur,

(h) le démontage de l'article d'avec la forme,

(i) l'application sur le revêtement de polymère en hydrogel d'un solution de l'agent tensioactif contenant également un silicone, et

(j) le chauffage du revêtement d'agent tensioactif conférant des propriétés permanentes de glissement au revêtement.